(19) Europäisches Patentamt
European Patent Office
Office européen des brevets

(11) EP 3 151 040 A1

(12) EUROPEAN PATENT APPLICATION

(43) Date of publication:
**05.04.2017 Bulletin 2017/14**

(51) Int Cl.:
***G02B 1/04*** (2006.01) ***G02C 7/04*** (2006.01)
***A61F 9/00*** (2006.01)

(21) Application number: **16179554.7**

(22) Date of filing: **20.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.12.2012 US 201261740599 P**

(62) Document number(s) of the earlier application(s) in accordance with Art. 76 EPC:
**13821910.0 / 2 936 215**

(71) Applicant: **CooperVision International Holding Company, LP**
**St. Michael (BB)**

(72) Inventors:
- **ZHU, Peter**
  **Pleasanton, CA 94588 (US)**
- **MORRIS, Carol Ann**
  **Pleasanton, CA 94588 (US)**
- **LUK, Andrew**
  **Pleasanton, CA 94588 (US)**
- **MALTSEVA, Inna**
  **Pleasanton, CA 94588 (US)**
- **BACK, Arthur**
  **Pleasanton, CA 94588 (US)**
- **ROGERS, Victoria**
  **Pleasanton, CA 94588 (US)**
- **KHONG, Kathleen**
  **Pleasanton, CA 94588 (US)**
- **ZHANG, Yun**
  **Pleasanton, CA 94588 (US)**
- **YE, Ying**
  **Pleasanton, CA 94588 (US)**

(74) Representative: **Fletcher, Matthew James Edwin**
**Abel & Imray**
**20 Red Lion Street**
**London WC1R 4PQ (GB)**

Remarks:
This application was filed on 14.07.2016 as a divisional application to the application mentioned under INID code 62.

# (54) ANTIMICROBIAL OPHTHALMIC LENSES

(57) Antimicrobial ophthalmic devices such as contact lenses, ocular inserts, ocular bandages, and intraocular lenses comprising an antimicrobially-effective amount of epsilon polylysine.

EP 3 151 040 A1

## Description

## BACKGROUND

The field of the disclosure is antimicrobial ophthalmic devices made from hydrogels and ε-polylysine.

ε-polylysine is a homopolymer of about 25-35 residues of L-lysine in which the epsilon-amino and carboxyl groups of L-Lysine are linked. It is a naturally-occurring polymer produced by *Streptomyces* species. It has broad-spectrum antimicrobial activity and has been widely used as a food preservative in Japan and as an additive in a variety of consumer products. Its use in contact lens care solutions has been described (see e.g. U.S. Pat. No. 6,187,264, and U.S. Pat. Publ. No. 2005/0074467). Antimicrobial hydrogels made from epsilon-poly-L-lysine-graft-methacrylamide have been reported (Zhou et al., Biomaterials 32 (2011) 2704-2712). Contact lens packages including a sealed receptacle that contains a contact lens made of a silicone hydrogel copolymer in a sterile solution which comprises a stabilizing agent which can form an ionic complex or hydrogen bond with the hydrogel copolymer, have been described in U.S. Pat. Publ. No. 2007/0149428. A packaging system and method for the storage of an ionic hydrogel lens that uses an aqueous packing solution which includes a phosphorylcholine polymer, and which further can include a buffering agent, have been described in U.S. Pat. Publ. No. 2009/0100801. Other background publications include U.S. Pat. Publ. No. 2012/0074352, U.S. Pat. Publ. No. 2011/0071091, U.S. Pat. Publ. No. 2005/0074467, U.S. Pat. Publ. No. 2004/0135967, U.S. Pat. No. 4,168,112, U.S. Pat. No. 7,282,214, U.S. Pat. No. 7,402,318, EP Pat. No. 1328303B1, and PCT Publ. No. WO94/13774.

## SUMMARY

In one aspect, the invention provides an antimicrobial contact lens immersed in a packaging solution and sealed in a package, said contact lens comprising a hydrogel and an antimicrobially-effective amount of epsilon polylysine (εPLL). Advantageously the contact lens comprises a first polymeric component which is a hydrogel and a second polymeric component which comprises the antimicrobially-effective amount of epsilon polylysine (εPLL). In a further aspect, the invention provides a sealed package containing an antimicrobial contact lens immersed in a packaging solution, said contact lens comprising a hydrogel and an antimicrobially-effective amount of epsilon polylysine (εPLL). Advantageously, the εPLL (or second polymeric component) is non-covalently attached to the hydrogel (or first polymeric component). In a yet further aspect, the invention provides a method of administering εPLL to the ocular tissue of a patient in need thereof, said method comprising administering the patient the contact lens of the invention. In another aspect, the invention provides a composition for use in the treatment or prophylaxis of a microbial infection comprising a hydrogel and εPLL. Advantageously, the composition is in the form of a contact lens of the invention.

## BRIEF DESCRIPTION OF THE DRAWING

**FIG. 1** depicts a set-up for testing biofilm formation on a contact lens.

## DETAILED DESCRIPTION

Antimicrobial ophthalmic devices are disclosed herein. The ophthalmic device comprises a hydrogel and an antimicrobially-effective amount of ε-polylysine (εPLL) non-covalently attached to the hydrogel. The εPLL is, or is a part of a polymeric component that is, distinct from a polymeric component that forms the hydrogel. Whilst the hydrogel polymeric component (first polymeric component) and the polymeric component that comprises the εPLL(second polymeric component) may be, and are advantageously, non-covalently attached to one another, for example by physical or electrostatic interactions, they are not covalently linked to one another and remain as separate distinct components of the contact lens. Contact lenses are exemplified herein, however other types of ophthalmic devices made from hydrogels, such as ocular inserts, ocular bandages, and intraocular lenses can be made in accordance with the present disclosure. The ophthalmic device is provided unworn (i.e. it is a new device, not having been previously used by a patient) sealed in a package, such as a blister package, glass vial, or other suitable container, containing a packaging solution in which the ophthalmic device is immersed.

The hydrogel can be manufactured by polymerizing a monomer mixture to form a polymerization product, and hydrating the polymerization product to obtain the hydrogel. As used herein, the term "monomer mixture" refers to a mixture of polymerizable monomers together with any additional ingredients, including non-polymerizable ingredients, which are subjected to polymerization conditions to form a polymerization product. The term "polymerization product" refers to a dry, i.e. non-hydrated, polymer which is then contacted with one or more liquids to hydrate and extract any unbound monomers or oligomers from the polymer and form the hydrogel. The term "monomer" refers to any molecule capable of reacting in a polymerization reaction with other molecules that are the same or different, to form a polymer

or copolymer. Thus, the term encompasses polymerizable pre-polymers and macromers, there being no size-constraint of the monomer unless indicated otherwise.

Methods of manufacturing hydrogel ophthalmic devices are well-known in the art. Exemplary methods are described in U.S. Pat No. 6,867,245, to Iwata et al., U.S. Pat. No. 8,129,442 to Ueyama et al., U.S. Pat No. 4,889,664 to Kindt-Larsen et al., U.S. Pat. No. 3,630,200 to Higuchi, and U.S. Pat. No. 6,217,896 to Benjamin. In the case of contact lenses, so-called "conventional hydrogels" are typically formed from a monomer mixture comprising a hydrophilic monomer such as 2-hydroxyethyl methacrylate (HEMA) or vinyl alcohol, optionally in combination with other monomers, and containing no siloxane (i.e. a molecule comprising at least one Si-O group). A silicone hydrogel is formed from a monomer mixture that comprises at least one polymerizable siloxane monomer. Examples of conventional hydrogels include etafilcon A, nelfilcon A, ocufilcon D, omafilcon A, omafilcon D and polymacon. Examples of silicone hydrogels include balafilcon A, comfilcon A, enfilcon A, lotrafilcon A, and senofilcon A. The above-mentioned hydrogels, and any other suitable hydrogel not specifically mentioned herein may be used in the antimicrobial ophthalmic device of the present disclosure. In a specific example, the antimicrobial ophthalmic device is a silicone hydrogel contact lens. In various examples, the silicone hydrogel contact lens is an extended-wear contact lens, which a patient wears continuously for at least 24 hours, 5 days, 7 days, or 14 days. In another example, the contact lens is a daily-wear lens, which a patient wears during the day and stores each night in a solution intended for contact lens storage. In yet another example, the contact lens is a daily-disposable lens, which is worn by a patient during waking hours, removed and discarded prior to sleep, and replaced by a new, unworn lens each day. Throughout this disclosure a reference to "examples", "an example", "one example" , "a specific example" or similar phrase, is intended to introduce a feature or features of the hydrogel, contact lens, εPLL, packaging solution, polymerizable composition, method of manufacture, etc. (depending on context) that can be combined with any combination of previously-described or subsequently-described examples (i.e. features), unless a particular combination of features is mutually exclusive, or if context indicates otherwise.

εPLL (CAS no. 28211-04-3) is commercially available typically as a homopolymer ranging from about 25 to about 35 lysine (LYS) residues. All fractions of the naturally-occurring homopolymer of εPLL may be used. Alternatively, a select fraction of the εPLL may be removed and discarded prior to use. For example, εPLL consisting of a homopolymer having at least 27 LYS residues, or at least 29 LYS residues, may be attached to the hydrogel. In another example, the εPLL attached to the hydrogel may consist of no more than 33 LYS residues, or no more than 31 LYS residues. In yet another example, the εPLL, or the εPLL portion of the second polymeric component, attached to the hydrogel consists of homopolymers ranging from 27 to 33 LYS residues. As used herein, the term εPLL also includes derivatives of εPLL provided that the derivatized form of the homopolymer retains antimicrobial activity. The second polymeric component may include εPLL or a derivative thereof incorporated into a larger compound. For example, prior to incorporation into the hydrogel, a chemical moiety may be linked to the εPLL to impart a desired property. In one example, the εPLL may be covalently attached to a hydrophobic moiety to improve its miscibility with a hydrophobic monomer mixture used in making the hydrogel or to slow its rate of release from the hydrogel. In another example, the εPLL may be linked to another polymer, providing a dual-functional block co-polymer, such as εPLL linked to a hydrophilic polymer that enhances the comfort of a contact lens while providing an antimicrobial effect. In other examples, the εPLL consists of an epsilon-L-lysine homopolymer, i.e. it is not derivatized. As used herein, the term "polymeric component that includes εPLL" includes a homopolymer of epsilon-L-lysine, a derivatized polymer of epsilon-L-lysine, a block co-polymer that includes an epsilon-L-lysine polymer and another polymer and a component that includes a non-polymeric portion (e.g. a hydrophobic moiety) linked to an epsilon-L-lysine polymer , for example, as a constituent of a polymeric component that is present in the hydrogel. It is to be understood that references to "εPLL" hereinafter are typically a shorthand for "polymeric component comprising εPLL". Whilst the term "εPLL" may in some contexts refer to homopolymers of epsilon-L-lysine or derivatives thereof only, where the context allows, the term "εPLL" is hereinafter to be understood as referring to both epsilon-L-lysine polymers and polymeric components that includes εPLL as defined above. Similarly references to "hydrogel" refer to a polymeric hydrogel component.

The contact lens comprises a hydrogel and an antimicrobially-effective amount of epsilon polylysine (εPLL). Typically, the εPLL is present in the hydrogel. Advantageously, εPLL is attached to the hydrogel by any non-covalent mechanism in which the εPLL maintains antimicrobial activity. The phrase "attached to" is not intended to limit the manner in which the ophthalmic device comprises the εPLL. For example, εPLL, is considered attached to a hydrogel if it is physically entangled within the polymer network of the hydrogel, and/or is taken up and retained in pores of the hydrogel by virtue of a concentration gradient between the hydrogel and the packaging solution into which the hydrogel is initially immersed, and/or is retained by the hydrogel due to hydrophobic interactions. As used herein, the term "non-covalently attached" includes attachment by electrostatic interactions, including ionic bonding between negatively charged groups present in the hydrogel and the positively charged amine groups present on the εPLL. The εPLL may, for example, be attached to the hydrogel by physical or electrostatic interactions. The εPLL need not be uniformly present throughout the hydrogel. For example, the εPLL can be present primarily on the surface of the hydrogel, or can be present at a greater concentration on the surface of the hydrogel, or can be present at a greater concentration in the bulk of the hydrogel. The polymeric component that includes the εPLL and the hydrogel component remain distinct when attached

to one another in the contact lens of the invention and are not linked to one another by covalent bonds, for example, as parts of a single molecule or integrated into the same cross-linked matrix.

The hydrogel optionally comprises negatively charged groups. Negatively charged groups ionically bind to the εPLL and facilitate the non-covalent attachment of the εPLL to the hydrogel. Advantageously, the hydrogel comprises negatively charged groups that ionically bind to the εPLL. In one example, the hydrogel comprises negatively charged groups to which primary amine groups on the εPLL ionically bind. A hydrogel with negatively charged groups may be prepared by including an anionic monomer in the monomer mixture used to make the polymerization product that is hydrated to form the hydrogel. Alternatively, negatively charged groups may be added after the polymerization product is formed, as described further below. Suitable negatively charged groups include carboxylate, phosphate, phosphonate, phosphonic, sulfonate, sulfate and sulfite groups. Hydrogels comprising carboxylate groups are particularly suitable for use in the present invention.

Anionic monomers suitable for use in monomer mixtures may comprise one or more carboxylate group, phosphate group, phosphonate group, phosphonic group, sulfonate group, sulfate group, sulfite group, and combinations thereof. Such groups are typically negatively charged at pH 7. Non-limiting examples of carboxylic acid-containing anionic monomers that can be used include (meth)acrylic acid, acrylic acid, itaconic acid, crotonic acid, cinnamic acid, vinylbenzoic acid, fumaric acid, maleic acid, monoesters of fumaric acid, and N-vinyloxycarbonyl-L-alanine; phosphate group-containing anionic monomers include 2-hydroxyethyl acrylate phosphate, 4-hydroxybutyl acrylate phosphate, and vinyl phosphate; and sulfonate group-containing monomers include styrene sulfonate, 2-acrylamido-2-methylpropane sulfonic acid, vinyl sulfonate, and sulfoethyl methacrylate. In one particular example, the carboxylic acid-containing anionic monomer is (meth)acrylic acid. The term "anionic monomer" also includes monomers that can undergo hydrolysis to provide a negative charge at about pH 7. For example, trimethysilyl methacrylate (TMSMA) may be included in a monomer mixture and polymerized. When the resulting polymerization product is hydrated, the trimethylsilyl group hydrolyses to generate methacrylic acid (i.e. the structure of a polymerized methacrylic acid monomer).

Advantageously, one or more anionic monomers is included in the monomer mixture in an amount to provide the hydrogel of the invention with an ionic content of from about 0.1% 0.3%, 0.5%, 1.0%, or 1.5% up to about 2.0%, 2.2%, 2.5%, or 3.0%. As used herein a % ionic content is determined by Formula I:

$$\sum (\boldsymbol{a_n}\, x\, \boldsymbol{b_n} / \mathbf{c_n})\ \mathrm{x}\ 89 = \%\ \text{ionic content} \qquad \mathbf{(I)}$$

where $\boldsymbol{a_n}$ is the weight percentage, as defined below, of ionic monomer **n** used in the monomer mixture, $\boldsymbol{b_n}$ is the number of negatively-charged groups on monomer **n** at pH 7 (for example, the number of carboxylate, phosphate, phosphonate, phosphonic, sulfonate, sulfate and sulfite groups in the monomer), and $\boldsymbol{c_n}$ is the molecular weight of ionic monomer **n.** If more than one anionic monomer is used in a monomer mixture, the % ionic content of the hydrogel is the sum of the % ionic content provided by each anionic monomer **n.** The weight percentage of the anionic monomer **n** in the monomer mixture is relative to the weight of all components of the monomer mixture that incorporate into the hydrogel. In other words, ingredients of the monomer mixture that do not incorporate into the final hydrogel product, such as diluents that are removed from the hydrogel during the manufacturing process, are not included in the weight percent determination. Formula I adjusts for differences in molecular weight and charge relative to (meth)acrylic acid, a commonly used anionic monomer in conventional hydrogel contact lenses, which has a molecular weight of 89 and one ionic group. Thus, for example, the ionic content of a hydrogel prepared from a composition that comprises 2.0 wt. % ofN-vinyloxycarbonyl-L-alanine (MW = 159, 1 ionic group) and no other anionic monomers is calculated as follows: (2.0 / 159) x (89) = 1.1 % ionic content. The ionic content of a hydrogel prepared from a composition that comprises 2.0 wt. % itaconic acid (MW = 130, 2 ionic groups) and no other anionic monomers is calculated as follows: (2.0 x 2 / 130) x 89 = 2.7 % ionic content. The ionic content of the hydrogel lens has effects the ability of a lens to uptake and release εPLL. We have found that sustained release of εPLL can be achieved by balancing the ionic content of the hydrogel of the lens to within the ranges described above.

Throughout this description, when a series of lower limit ranges and a series of upper limit ranges are provided, all combinations of the provided ranges are contemplated as if each combination were specifically listed. For example, in the above listing of ionic content percentages, all 20 possible percent ionic content ranges are contemplated (i.e. 0.1-2.0%, 0.3-2.0%... 1.5%-2.5%, and 1.5%-3.0%). Further, throughout this disclosure, when a series of values is presented with a qualifier preceding the first value, the qualifier is intended to implicitly precede each subsequent value in the series unless context dictates otherwise. For example, for the values listed above, it is intended that the qualifier "from about" implicitly precedes the values 0.3, 0.5, 1.0, and 1.5, and the qualifier "up to about" implicitly precedes the values 2.2, 2.5, and 3.0. In a specific example, the ionic content of the hydrogel is in the range of 1.5% to 2.2%, or 1.6% to 2.0%. We have found, surprisingly, that hydrogel contact lenses having an ionic content in this range can sustain release of an antimicrobially effective amount of εPLL for at least 7 days.

Alternatively, or in addition to including anionic monomers in the monomer mixture, negatively charged groups may be incorporated into a hydrogel after the monomer mixture is polymerized. For example, plasma treatment can be used to attach anionic monomers onto the surface of the polymerization product or hydrogel (see e.g. U.S. Pat. No. 4,143,949 to Chen et al. and U.S. Publ. No. 2008/0245474 to Claude et al.). In another method, a monomer comprising a reactive functional group, such as a hydroxyl group or an amine group, is included in the monomer mixture. The resulting polymerization product may be subsequently conjugated via the reactive functional groups to a negatively charged compound.

εPLL can attach to a hydrogel simply by immersing the polymerization product or hydrogel in a packaging solution that comprises εPLL. If the hydrogel contains negatively charged groups, as described above, it can ionically bind with primary amine groups of the εPLL. Some hydrogels without negatively charged groups can also take up an antimicrobially-effective amount of εPLL from a packaging solution. For example, due to the εPLL concentration gradient between the packaging solution and hydrogel, an effective amount of εPLL may be taken up by the hydrogel and retained due to hydrophobic or other physical interactions. For example, we have found that enfilcon A, which has no negatively charged groups, can take up an antimicrobially-effective amount of εPLL from PBS comprising 500 ppm εPLL. We have also found that omafilcon A, a hydrogel made from a monomer mixture comprising HEMA and a zwitterionic monomer, methacryloyloxyethyl phosphorylcholine (MPC), takes up an antimicrobially-effective amount of εPLL from PBS comprising 500 ppm εPLL. The hydrogel optionally comprises phosphorylcholine groups. Zwitterionic phosphorylcholine groups ionically bind to εPLL and facilitate the non-covalent attachment of the εPLL to the hydrogel. Advantageously, the hydrogel comprises phosphorylcholine groups that ionically bind to the εPLL.

In one example, the packaging solution, just prior to contact with the hydrogel or polymerization product, comprises εPLL in amounts of at least 50 ppm, 100 ppm, 150 ppm, 200 ppm, 250 ppm, or 300 ppm up to about 600 ppm, 800 ppm, 1000 ppm 1500 ppm or 2000 ppm. As used herein, a solution that comprises 500 ppm εPLL contains 500 μg/ml εPLL. While εPLL concentrations in these ranges will be highly effective against ocular pathogens most often implicated in contact lens-induced bacterial keratitis, concentrations greater than 2000 ppm are non-toxic and non-irritating to ocular tissue and can be used in examples where a higher concentration of εPLL is desired. After the hydrogel or polymerization product is immersed in the packaging solution, some, most or all of the εPLL non-covalently attaches to the hydrogel, thereby reducing the concentration of εPLL in the solution. After the hydrogel is immersed in the packaging solution, the package is sealed and optionally sterilized. Suitable sterilization methods include autoclaving, gamma radiation, e-beam radiation, ultraviolet radiation, etc. In some examples, the hydrogel and packaging solution may be manufactured and combined using sterile conditions, such that a post-packaging sterilization step is unnecessary.

We have found that by decreasing the ionic strength of the packaging solution from what is conventionally used for contact lenses, uptake and/or retention of εPLL by hydrogels can be significantly increased, resulting in enhanced bioactivity. For example, omafilcon A contact lenses, when packaged and autoclaved together with 500 ppm εPLL in PBS having an ionic strength of about 0.2, take up about 5 μg εPLL/lens. When tested in the *in vitro* bioactivity assay substantially as described in Example 5 below using a 24-hour incubation with $10^4$ bacterial inoculum, the lenses resulted in a complete kill of *Pseudomonas aeruginosa* (PA), less than a one log kill of *Staphylococcus aureus* (SA), and no detectable kill of *Serratia marcescens* (SM). In contrast, the same omafilcon A lenses packaged and autoclaved in TRIS buffer with 2% sorbitol, which has an ionic strength of about 0.02, took up about 120 μg εPLL/lens and resulted in about a 4-log kill of SM. Thus, in various examples, the packaging solution has an ionic strength of less than about 0.15, 0.10, or 0.05 as calculated by the equation:

$$I = \frac{1}{2}\sum_{i=1}^{n} c_i z_i^2$$

where $c_i$ is the molar concentration of ion i ($mol \cdot dm^{-3}$), $z_i$ is the charge number of that ion, and the sum is taken over all ions in the packaging solution.

To reduce ionic strength while maintaining proper osmolality in the range of about 200, 250, or 270 mOsm/kg up to about 310, 350, or 400 mOsm/kg, sodium chloride, which is commonly used as a tonicity agent in contact lens packaging solutions, can be replaced with a non-electrolyte tonicity agent, such as sorbitol, as indicated above. Other non-electrolyte tonicity agents that can be used in the packaging solution include mannitol, glucose, glycerol, propylene glycol, xylitol, and inositol. Additionally or alternatively, the ionic strength of a packaging solution can be reduced by substituting the phosphate or borate buffer used in conventional contact lens packaging solutions with a lower ionic strength buffer such as TRIS and/or tricine. Example 8 below further demonstrates that lowering the ionic strength of a packaging solution comprising εPLL can result in increased uptake of εPLL by the hydrogel.

In some examples, the packaging solution consists, or consists essentially, of an aqueous solution of a buffer, a tonicity agent, and optionally εPLL. In other examples, the packaging solution contains additional agents such as one

or more additional antimicrobial agents, a comfort agent, a hydrophilic polymer, or a surfactant or other additive that prevents the lens from sticking to the container. The packaging solution typically has a pH in the range of about 6.8 or 7.0 up to about 7.8 or 8.0.

Another way by which εPLL can be attached to a hydrogel is by including the εPLL in the monomer mixture. After polymerization of the monomer mixture the εPLL is entwined within the polymer network of the hydrogel and, because it is non-covalently attached to the hydrogel, can migrate through the pores of the hydrogel to the surface where it can provide an antimicrobial effect.

εPLL can also be attached to a hydrogel by immersing a polymerization product into an aqueous solution comprising εPLL under conditions and for a time sufficient for the polymerization product to hydrate and take up an antimicrobially-effective amount of εPLL. Alternatively, the polymerization product may be hydrated to form a hydrogel, and then immersed in a solution comprising εPLL under conditions and for a time sufficient for the hydrogel to take up an antimicrobially-effective amount of εPLL. The resulting hydrogel may be packaged in a packaging solution that is either free of εPLL or contains additional εPLL.

An "antimicrobially-effective amount of εPLL", as used herein, is an amount of εPLL that results in at least a two log kill of *Pseudomonas aeruginosa* (PA) compared to a control hydrogel when tested after 24 hours incubation with $10^4$ CFU PA (i.e. low inoculum) using an *in vitro* bioactivity assay. As used herein, an *"in vitro* bioactivity assay" means an assay substantially as described in Example 5 below and a "control hydrogel" means a hydrogel that does not comprise εPLL but is otherwise substantially identical to the εPLL-containing hydrogel being tested in the *in vitro* bioactivity assay. Further, a reference herein to a "log kill" against an organism means the log kill obtained using the *in vitro* bioactivity assay after a 24-hour incubation with $10^4$ CFU of the organism, unless indicated otherwise. In some examples, the εPLL-containing hydrogel results in at least a four log kill of PA. In further examples, the εPLL-containing hydrogel results in at least a four log kill of PA when tested in the *in vitro* bioactivity assay after a 24-hour incubation with $10^7$ CFU of the organism (i.e. high inoculum). In yet further examples, live PA is not detectable on the εPLL-containing hydrogel when tested in the *in vitro* bioactivity assay after a 24-hour incubation with a low inoculum of PA or even a high inoculum of PA, whereas the control lens supports a PA growth. In such examples, the antimicrobial hydrogel is said to result in a complete kill of PA. In various examples, the antimicrobial hydrogel results in at least a one log kill or two log kill of *Serratia marcescens* (SM) and/or *Staphylococcus aureus* (SA) using a low inoculum, or even a high inoculum. As used herein, SA, SM, and PA refer to the strains of these bacteria listed in Table 3 of Example 5, or equivalent strains that have substantially the same sensitivities to antimicrobial agents as the strains listed in Table 3.

The hydrogel may retain an antimicrobially-effective amount of εPLL after being subjected to the *in vitro* εPLL release conditions described in Example 4 below, hereinafter referred to as an *"in vitro* release assay". As used herein, a time of εPLL release is in reference to the *in vitro* release assay. Thus, for example, a hydrogel that is said to result in a two log kill of PA after 24 hours of εPLL release means that the hydrogel, when subjected to the *in vitro* εPLL release assay for 24 hours and thereafter tested in the *in vitro* bioactivity results in a two log kill of PA. In some examples, the hydrogel results in at least a two log kill of PA, SM, and/or SA after 48 hours, 72 hours, 96 hours, 120 hours, or even 168 hours of εPLL release. While the antimicrobial ophthalmic devices described herein are suitable for all modalities of wear, hydrogels that retain an antimicrobially-effective amount of εPLL after 48 hours of εPLL release can be particularly suitable for extended wear. Optionally, a biofilm assay substantially as described in Example 6 below can be used to measure the antimicrobial effectiveness of a hydrogel after εPLL release. In various examples, an antimicrobial hydrogel comprises at least about 0.05 wt.%, 0.1 wt.%, 0.2 wt.%, 0.5 wt.%, or 1.0 wt.% εPLL and up to about 2.0 wt.%, 3.0 wt.% or 4.0 wt.% εPLL, wherein the wt.% of εPLL is based on the total dry weight of the hydrogel. For hydrogel contact lenses, an antimicrobially-effective amount of non-covalently attached εPLL is typically at least about 5 μg, 10 μg, 25 μg, 50 μg, 100 μg, or 150 μg εPLL and up to about 300 μg, 400 μg, 500 μg or 600 μg. While an amount of εPLL within these ranges will normally be highly effective against ocular pathogens most often implicated in contact lens-induced bacterial keratitis, amounts greater than 600 μg are non-toxic and non-irritating to ocular tissue and can be used in examples where a higher amount of εPLL is desired.

In some examples, the hydrogel has a release profile in which εPLL release is sustained for at least 2 hours, resulting in sustained antimicrobial activity. As used herein, reference to an amount of εPLL released refers to the amount of εPLL released from a hydrogel for a given duration of time as tested in an *in vitro* release assay. A hydrogel is said to exhibit "sustained release" of εPLL for at least a given duration of time if there is a significant increase in amount of εPLL between the end of that given duration of time and the next time duration of an *in vitro* release assay. For example, if a hydrogel releases 30 μg εPLL between 0-2 hours, and releases an additional 10 μg εPLL between 2-4 hours, as determined using the *in vitro* release assay, the hydrogel is said to sustain release of εPLL for at least 2 hours. In some examples, the hydrogel sustains release of εPLL for at least 4 hours, 6 hours, 8 hours, or 24 hours. Typically, the initial rate of εPLL release is relatively high. This is advantageous in that it provides a burst of εPLL release upon initial insertion of the ophthalmic device when introduction of pathogens is most likely to occur due to handling. The ionic content of the hydrogel and the concentration of εPLL in the packaging solution can be balanced to provide desirable εPLL release profiles. In one example, the hydrogel releases at least 1 μg, 5 μg, 10 μg, 20 μg, 30 μg or 40 μg εPLL and up to about

60 μg, 80 μg, or 100 μg εPLL in 2 hours. In a further example, the hydrogel exhibits sustained release of εPLL, releasing the above-mentioned amounts of εPLL by 2 hours and releasing an additional 1 μg, 5 μg, 10 μg or 20 μg and up to about 30 μg, 40 μg, or 60 μg of εPLL between 2 and 4 hours. In some examples, the hydrogel has a release profile in which εPLL release is sustained for at least 12 hours, i.e. the hydrogel exhibits significant εPLL release between the 12 and 24 hour time points as determined using the *in vitro* release assay. Thus, a contact lens comprising the hydrogel may continue to have an antimicrobial effect when worn overnight. Surprisingly, we have found that hydrogel contact lenses having an ionic content of about 1.6% to about 2.0% packaged in a solution comprising about 200 to about 1000 ppm εPLL can be subjected to *in vitro* release for 7 days and remain antimicrobially-effective. This is demonstrated in Example 6.

The hydrogel may optionally comprise a second antimicrobial agent to enhance its antimicrobial properties. We have found that incorporating a second antimicrobial agent into the hydrogel can result in a synergistic antimicrobial effect. For example, an ionic silicone hydrogel contact lens comprising about 10 μg polyquaternium-1 (PQ-1), and no other antimicrobial agent, has no significant activity against SA or SM, as determined using a bioactivity assay after a 24 hour incubation with $10^7$ inoculum (described in Example 7). The same type of contact lens comprising about 200 μg εPLL and no other antimicrobial agent, results in about a 2-log kill of SA and about a 1.5 log kill of SM. When the contact lens comprises both 10 μg PQ-1 and 200 μg εPLL, it may result in about a 2.5 log kill of SA and a 4 log kill of SM. The contact lens is said to comprise an amount of PQ-1 that synergistically increases its antimicrobial activity against SA and SM because the antimicrobial activity against these organisms is significantly greater than the combined activity of the lenses comprising εPLL and no PQ-1 with the lenses comprising PQ-1 and no εPLL. The *in vitro* biofilm assay of Example 6 can also be used to test synergistic increases in antimicrobial activity provided by a second antimicrobial agent. Examples of other second antimicrobial agents that may be incorporated into the hydrogel include other antimicrobial peptides (e.g. defensin), polyhexamethylene biguanide (PHMB), alexidine, and the like. In a specific example, the hydrogel comprises an anionic component, εPLL, and an amount of a second antimicrobial agent to synergistically increase antimicrobial activity against SA or SM as determined by an *in vitro* biofilm assay.

The antimicrobial ophthalmic devices described herein are non-irritating and non-toxic to the ocular tissue which they are intended to contact. For example, contact lenses, which contact the anterior segment of the eye, are not irritating to the corneal epithelium or conjunctiva. *In vitro* cytotoxicity testing in accordance with ISO 10993 (ANSI / AAMI / ISO 10993-5: Biological evaluation of medical devices - Part 5: Tests for in vitro cytotoxicity, 1999) is recognized as predictive *of in vivo* biocompatibility of ophthalmic devices. Briefly, in the case of contact lenses, the lenses are removed from their packaging solution and, without rinsing or washing, are placed by direct contact along with 0.8 mL MEM with 5% calf serum on a confluent monolayer of L929 cells. After incubation at 37° C for 24 hours with 5% $CO_2$, cytoxicity is scored by zone of cell death under or around the lenses according to cytopathic scoring methods per USP Guidelines (USP <87> Biological Reactivity; United States Pharmacopeia 32 / National Formulary 27, United States Pharmacopeial Convention, Rockville, MD, 2009). As used herein, a hydrogel is considered "ophthalmically-acceptable" if it scores a cytopathic grade of two or less using this assay. Desirably, the ophthalmic device will score a cytopathic grade of one or zero and provide at least a four log kill of *Pseudomonas auruginosa* when tested in an *in vitro* bioactivity assay using a $10^4$ inoculum and a 24-hour incubation.

We have found that incorporation of an antimicrobially-effective amount of εPLL into a contact lens can be achieved without adversely impacting the physical properties of the lens, such as optical clarity, modulus, wettability, etc. For example, the contact lenses described herein are optically clear, having a light transmittance between 380 nm to 780 nm of at least 93%, 95% or 97% as measured in accordance with ISO 18369.

In view of the foregoing, it will be appreciated that conventional manufacturing methods can be used to manufacture the antimicrobial ophthalmic devices described herein. Thus, one aspect of the present disclosure is a method of manufacturing an unworn antimicrobial contact lens, said method comprising: polymerizing a monomer mixture to provide a lens-shaped polymerization product; optionally hydrating the polymerization product to form a hydrogel; immersing the hydrogel or polymerization product in a package containing a packaging solution; and sealing the package, wherein an antimicrobially effective amount of εPLL is non-covalently attached to the hydrogel. In one example, εPLL is present in the packaging solution into which the hydrogel is immersed. Thus, in one aspect of the present disclosure is a method of manufacturing a packaged antimicrobial hydrogel contact lens including the step of bringing a lens-shaped polymerization product or a hydrogel contact lens into contact with a packaging solution that comprises εPLL. The contacting of a lens-shaped polymerization product with the packaging solution may, for example, result in both hydration of the contact lens and attachment of εPLL to the resulting hydrogel. The packaging solution, which is brought into contact with the lens-shaped polymerization product or hydrogel contact lens, may, for example, include from about 50 ppm to about 10,000 ppm, especially from about 100 ppm to about 600 ppm εPLL. In another example, the εPLL is a component of the monomer mixture. In yet another example, the εPLL is attached to the hydrogel in a post-polymerization process step, either to the unhydrated or hydrated polymerization product, prior to immersion in the packaging solution. The manufacturing method may optionally comprise the additional step of sterilizing the sealed package, for example, by autoclave. Generally, the final manufactured product includes at least a sealed container containing an unused contact

lens immersed in an aqueous lens packaging solution in accordance with the above-described examples. The sealed container may be a hermetically sealed blister-pack, in which a concave well containing a contact lens is covered by a metal or plastic sheet adapted for peeling in order to open the blister-pack. The sealed container may be any suitable inert packaging material providing a reasonable degree of protection to the lens, such as a plastic material such as polyalkylene (e.g., polyethylene or polypropylene), PVC, polyamide, and the like.

In the case of contact lenses intended for daily wear, in which the lens is removed and stored in a multi-purpose contact lens care solution (MPS) overnight, the anti-microbial activity of the lens may be replenished or enhanced by one or more antimicrobial agent present in the MPS that incorporates into the contact lens during the overnight storage. Examples of such antimicrobial agents include εPLL (to replenish εPLL released from the contact lens during the day), PQ-1, PHMB, alexidine, and the like. Thus, methods are provided herein for replenishing or enhancing antimicrobial activity of the antimicrobial contact lenses described herein after they have been worn. The method comprises storing the worn contact lens in a multi-purpose contact lens care solution (MPS) that comprises additional εPLL and/or a second antimicrobial agent, wherein additional εPLL and/or second antimicrobial agent incorporates into the contact lens during the storage. In a specific example, the contact lens comprises an anionic component that ionically binds the εPLL and/or second antimicrobial agent. In a further example, the contact lens is a daily wear silicone hydrogel contact lens having an ionic content of about 1% to about 2% and the method comprises overnight storage of the contact lens in MPS comprising PQ-1 or εPLL.

The composition and lenses of the invention comprising a hydrogel and εPLL may be used in the treatment or prophylaxis of a microbial infection , especially PA, SM, SA and other microbial infection described herein or which may be treated using εPLL. The composition and lenses of the invention may be especially suited for the treatment or prophylaxis of a microbial infection in the ocular tissue, and/or reducing the risk of adverse events in a patient to whom the composition or lens of the invention is administered. It will be appreciated that ophthalmic devices manufactured from the above-antimicrobial hydrogels, and as further detailed in the examples section below, can be used in a method of reducing risk of microbially-driven adverse events in a patient, as determined using a clinical study. Examples ofmicrobially-driven adverse events include contact lens acute red eye (CLARE), contact lens peripheral ulcer (CLPU), infiltrative keratitis (IK) and microbial keratitis (MK). In one example, the ophthalmic device is a contact lens that results in an average reduction of adhered pathogenic ocular bacteria of at least 50%, 80% or 90% adhered to the lens compared to a control lens that lacks εPLL but is otherwise substantially identical to the test lens, where the reduction of adhered ocular pathogenic bacteria is determined using a VITEK® 2 Microbial Identification System (bioMérieux SA) at the conclusion of a clinical study. In one example, the study compares the number of adhered pathogenic ocular bacteria on test and control lenses where the lenses are worn daily for one or more weeks (e.g. 7, 14, 21, 28 etc.) and stored each night in MPS. In another study, the lenses are worn continuously by the subjects for at least 24 hours, 48 hours, 5 days, 7 days, 14 days or 30 days.

The following Examples illustrate certain aspects and advantages of the present invention, which should be understood not to be limited thereby.

**Example 1: Preparation of Anionic Silicone Hydrogel Contact Lenses**

Six silicone hydrogel formulations, A-F, were made by weighing and mixing together the chemicals listed in Table 1 below in the relative parts (by weight) indicated and filtered using a 0.2 - 5.0 micron filter. The mono-functional siloxane listed in the Table 1 has structure II shown below. Methods of making this siloxane monomer are described in U.S. Pat. No. 8,168,735 to Ichinohe.

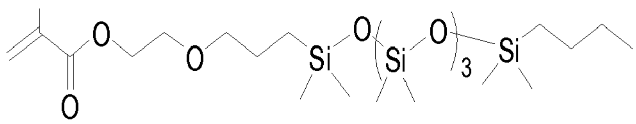

II.

The bi-functional siloxane macromer listed in Table 1 has structure III shown below, wherein n is about 90, m is about 5 and p is about 7. 0. Methods of making this macromer are described in U.S. Pat. No. 8,129,442 to Ueyama et al.

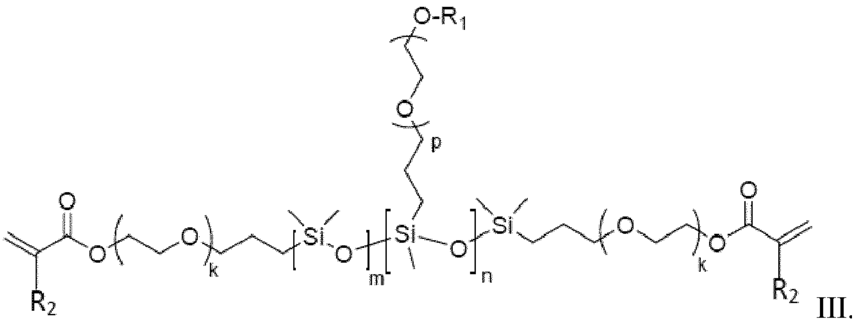

III.

**Table 1**

| Chemical | Parts by wt. | | | | | |
|---|---|---|---|---|---|---|
| Formulation # | A | B | C | D | E | F |
| (meth)acrylic acid | 0 | 1.4 | 1.8 | 2.4 | 3.0 | 1.8 |
| mono-functional siloxane monomer | 26 | 26 | 26 | 26 | 26 | 29 |
| bi-functional siloxane macromer | 10 | 10 | 10 | 10 | 10 | 8 |
| N-vinyl-N-methylacetamide | 45 | 45 | 45 | 45 | 45 | 45 |
| methyl methacrylate | 12 | 12 | 12 | 12 | 12 | 8 |
| 1,4-butanediol vinyl ether | 5 | 5 | 5 | 5 | 5 | -- |
| diethylene glycol vinyl ether | -- | -- | -- | -- | -- | 5 |
| ethylene glycol methyl ether methacrylate | -- | -- | -- | -- | -- | 6 |
| ethylene glycol methacrylate | 2 | 2 | 2 | 2 | 2 | -- |
| ethylene glycol dimethacrylate | -- | -- | -- | -- | -- | 0.6 |
| triethylene glycol dimethacrylate | 1.2 | 1.2 | 1.2 | 1.2 | 1.2 | -- |
| triethyleneglycol divinyl ether | 0.2 | 0.2 | 0.2 | 0.2 | 0.2 | 0.1 |
| Norbloc (CAS no. 96478-09-0) | -- | -- | -- | -- | -- | 1.7 |
| Diphenyl (P-vinylphenyl) phosphine (CAS no. 40538-11-2) | 0.5 | 0.5 | 0.5 | 0.5 | 0.5 | 0.5 |
| Reactive Blue 247 (CAS Reg. No. 109561-07-1) | -- | -- | -- | -- | -- | 0.01 |
| Vazo-64 (CAS reg. No. 78-67-1) | 0.5 | 0.5 | 0.5 | 0.5 | 0.5 | 0.5 |
| 2-Allyloxy ethanol | -- | -- | -- | -- | -- | 0.8 |

The resulting polymerizable monomer mixtures were cast molded in polypropylene contact lens mold assemblies and thermally cured in a nitrogen oven using conventional methods. Each cured lens was removed from its mold and hydrated and washed using multiple exchanges of deoinized water to remove unreacted and partially reacted components from the hydrogel.

**Example 2: *In Vitro* Uptake Assay**

εPLL at a concentration of 25% in water (Chisso Corporation, Tokyo, Japan) was used as a starting εPLL material in the following examples. Lenses of Formulation F prepared according to Example 1 were transferred to 6 ml glass vials containing either 1.2 ml PBS (control lens) or 1.2 ml 500 ppm εPLL in PBS. Unless indicated otherwise, references herein to PBS mean 29 mM PBS at pH 7.5 with an ionic strength of about 0.21 (0.78 wt.% NaCl, 0.05 wt.% sodium phosphate monobasic, and 0.36 wt.% sodium phosphate dibasic). The vials were sealed and autoclaved at 120° C for 30 minutes. Additionally, vials containing 1.2 ml 500 ppm εPLL in PBS with no lens (control vial) were also autoclaved. The amounts of εPLL present in the post-autoclave solution of the test lens vial and in the control vial were determined by cationic size exclusion chromatography using a sample injection volume of 20 μl, an Eprogen CATSEC300 5μ

250x4.6MM , at room temperature, and a flow rate of 1.0 ml/min using 0.2M NaCl/0.1% TFA in $H_2$0 isocratically.

The amount of εPLL taken up by the lenses was calculated by subtracting the amount of εPLL present in the post-autoclave solution of the test lens vial from the amount of εPLL present in the control vial. The average uptake was about 200 μg εPLL/lens.

**Example 3: Affect of ionic content of contact lens on uptake of εPLL**

Three lenses of each of Formulations A, and C-E prepared according to Example 1 were used in this study. The lenses were immersed in 1.2 ml of 500 ppm εPLL in PBS for 48 hours as described in Example 3. Excess solution was removed from each lens by gently blotting with an absorbent tissue. Each lens was immersed in an individual well of a 12-well plate containing 1.2 ml of 500 ppm εPLL in PBS. The plates were shaken for 48 hours at 100 rpm at a temperature of 25±2° C. The amount of εPLL taken up by each lens was calculated by subtracting the final concentration of εPLL from the initial concentration and multiplying by 1.2 (ml PBS). Formulation A lenses took up about 5 μg εPLL, C lenses took up about 220 μg εPLL, D lenses took up about 340 μg εPLL, and E lenses took up about 420 μg εPLL.

**Example 4: *In Vitro* Release Assay - Affect of IonicContent on εPLL Release**

Lenses of Formulations B-E prepared according to Example 1 were used in this study. Excess solution was removed from each lens by gently blotting with an absorbent tissue. Each lens was immersed in 1ml of ISO 10344 standard saline solution (0.83% sodium chloride, 0.0467% sodium phosphate monobasic, and 0.4486% sodium phosphate dibasic) in a well of a 12-well plate and covered. The plates were shaken at 100 rpm at 37±2° C. At 2, 4, 6, 8, 12, and 24 hours, the solution was removed from each well and replaced with 1ml of fresh ISO 10344 standard saline solution. HPLC was used to determine the amount of εPLL released from each lens. Table 2 shows the average cumulative amount (in μg) of εPLL released from the lens at each time point as well as the cumulative percentage of εPLL released relative to total amount of εPLL taken up by the lens.

**Table 2**

| | B | | C | | D | | E | |
|---|---|---|---|---|---|---|---|---|
| Time (hr) | μg | % | μg | % | μg | % | μg | % |
| 2 | 28±3 | 24 | 27±4 | 13 | 17±1 | 5 | 13±3 | 3 |
| 4 | 48±2 | 28 | 40±8 | 20 | 34±5 | 11 | 20±2 | 5 |
| 6 | 57±3 | 43 | 60±13 | 29 | 50±5 | 16 | 20±2 | 5 |
| 8 | 58±2 | 50 | 67±6 | 33 | 50±4 | 16 | 20±2 | 5 |
| 12 | 58±2 | 50 | 81±6 | 40 | 50±4 | 16 | 20±2 | 5 |
| 24 | 62±6 | 53 | 88±5 | 43 | 50±4 | 16 | 21±2 | 5 |

Only the lenses of Formulation C, which had 1.7% ionic component (provided from (meth)acrylic acid) sustained release of the εPLL for the entire duration of the assay. In contrast, Formulation B lenses, with 1.35% ionic content, released about the same amount of εPLL during for the first 6 hours, but had little, if any release after 6 hours. Interestingly, lenses of Formulation D, which had 2.3% ionic component and took up about 50% more εPLL than Formulation C lenses, released less εPLL than Formulation C lenses. As with Formulation B lenses, there was little, if any, release of εPLL after 6 hours. Even more striking, Formulation E lenses, which had 2.85% ionic content, and took up about twice as much εPLL as did Formulation C lenses, released the least amount of εPLL at the 2 and 4 hour time points, and release little if any εPLL after 4 hours. The results show that sustained release of εPLL can be achieved by balancing the ionic content of the lens.

To use the *in vitro* release assay to test for εPLL release beyond the 24 hour time point, the saline solution is removed and replaced every 24 hours (i.e. at 48 hours, 72 hours, 96 hours, etc.) and the plates shaken at 100 rpm at 37±2° C, as described above.

**Example 5: *In* Vitro Bioactivity Assay --**

**Preparation of Bacterial Suspensions:** Cultures were prepared from growing a single colony of each of the bacterial species shown in Table 3 below in 50 ml. trypticase soy broth (TSB) overnight at 37 °C on a rotary shaker. 1 ml of each culture was centrifuged, and the bacterial pellet resuspended in 1.0 ml of the diluent shown in Table 3, where

PBST is 19 mM PBS at pH 7.3 (0.83 wt.% NaCl, 0.03 wt.% sodium phosphate monobasic, and 0.24 % sodium phosphate dibasic) with 0.05% Tween. For each bacterial species, a suspension of approximately $10^8$ CFU/mL was prepared by diluting the bacterial suspension to achieve the optical density indicated in Table 3.

**Table 3.**

| Bacterial Species | Strain | $OD_{660}$ | Media/Diluent |
|---|---|---|---|
| *Pseudomonas aeruginosa* (PA) | ATCC 9027 | ~0.1 | PBS with 0.05% Tween (PBST) |
| *Serratia marcescens* (SM) | ATCC 6538 | ~0.2 | 0.05% TSB in PBST |
| *Staphylococcus aureus* (SA) | ATCC 13880 | ~0.3 | 2.0 % TSB in PBST |

Each suspension was further diluted to approximately$10^4$ CFU/mL (low inoculum) or $10^7$ CFU/mL (high inoculum) using the above diluents to make the bacterial inoculums for testing bioactivity of lenses.

**Testing Bioactivity of Lenses:** Each lens was rinsed in 2.5 ml sterile PBS for a few seconds, and transferred into an individual well of a 24-well plate containing 1.0 ml of bacteria inoculum. The plates were incubated at 37°C with gentle shaking. The original inoculums were incubated for the duration of the experiment to confirm the concentrations at each sample recovery time point. At the time point tested, each lens was removed from its well and transferred to a well of a 12-well plate containing 2.5 mL of sterile PBST. The plate was gently swirled gently for about 30 seconds. This step was repeated once for each lens. Such 2x wash step is referred to herein as the bacterial wash step.

**Recovery of Bacteria:** Each washed lens was placed in a microcentrifuge tube containing 1 ml of Dey-Engley (DE) neutralizing broth and adhered bacteria were removed by a combination of sonication for about 2 minutes and vortexing for about 10 minutes. Serial dilutions were made for each recovered cell suspension using DE neutralizing broth and suitable dilutions were plated onto TSA. Plates were incubated overnight at 37° C and CFUs were counted.

**Conversion of CFU counts to live bacteria recovered per lens:** The CFU for each plate was multiplied by the dilution factor (DF) as well as the plating dilution factor (PDF). The total CFUs recovered for a given sample were then converted to the log 10. To calculate the log kill of an antimicrobial lens, the log of CFU/lens of the antimicrobial lens is subtracted from that of the control lens. For example, if the mean log10 value of an antimicrobial lens is 1.05 and the mean log10 value of an otherwise identical control lens lacking the active antimicrobial agent is 5.52, the log kill is 5.52-1.05 = 4.47.

**Example 6: *In Vitro* Biofilm Assay -- εPLL-containing ionic lenses exhibit prolonged antimicrobial activity**

**Preparation of Washed Lenses:** εPLL-containing lenses of Formulation F, prepared as described in Example 2 were removed from their vials and placed into new vials containing 5 ml of PBST (release media). The vials were placed in a shaker at 37°C. RP-HPLC as described in Example 2 was used to determine the amount of εPLL in the release media at various time points. At each time point, the lenses were moved into fresh release media, either 5 ml (at 0, 6, and 24 hr) or 1 ml (all time points after 24 hr). The control lenses (i.e. without εPLL) were also tested to confirm the absence of interfering peaks from released material other than εPLL Table 4 shows the average cumulative amount (in μg) of εPLL released from the lens at each time point tested as well as the cumulative percentage of εPLL released relative to total amount of εPLL taken up by the lens.

**Table 4:**

| Time (hr) | Vol | μg | % |
|---|---|---|---|
| 0 | 5 ml | -- | -- |
| 6 | 5 ml | 85 ± 8 | 39 ± 4 |
| 24 | 5 ml | 115 ± 6 | 52 ± 3 |
| 48 | 1 ml | 120 ± 6 | 55 ± 3 |
| 72 | 1 ml | 123 ± 6 | 56 ± 3 |
| 96 | 1 ml | 126 ± 7 | 57 ± 4 |
| 168 | 1 ml | 129 ± 7 | 59 ± 3 |
| 192 | 1 ml | 130 ± 8 | 59 ± 4 |
| 216 | 1 ml | 132 ± 8 | 60 ± 4 |

Approximately 50% of the εPLL was released during the first 24 hours. The release of εPLL tapered off noticeably after 48 hours. Between days 7 and 8 only about 1 μg εPLL was released, and the lenses retained about 70 μg of εPLL.

**Bacterial Adhesion and Wash:** Lenses at the 0 hour time point and from the 168 hour time point were used to test whether they supported biofilm formation. The lenses were rinsed for 5 seconds in 2.5 mL of sterile PBS then transferred into an individual well of a 24-well plate containing 1.0 mL of bacterial inoculum at approximately $10^7$ CFU/mL, prepared as described in Example 5. The plates were incubated for 2 hours on a 37°C with gentle rotary agitation. The remaining inoculum, i.e. not incubated with lenses, was also incubated on the shaker for the duration of each experiment to determine the cfu/mL of the inoculum at t=2 hr and t=24 hr. After 2 hours incubation each lens was washed using the bacterial wash step described in Example 5 then placed on an insert of a 24-well plate for biofilm formation as described below. The inoculum concentration (at t=0 hr and 2 hr) was confirmed by serial dilution, plating onto TSA, incubating overnight at 37° C and colony counting.

**Biofilm Formation/Growth:** Custom-made mushroom-shaped stainless steel inserts, depicted in FIG. 1, were placed in each well of a 24-well plate. The cap portion of each insert has a diameter of approximately 14 mm and a curvature to match the posterior surface of a contact lens. The height of each insert is approximately 11 mm. One mL of sterile media/diluent was added to each well. Each preloaded, washed lens (as described above) was placed on the top (cap portion) of an insert, convex side up. The dimensions of the inserts and the volume of the media are such that the periphery of the placed lens is in contact with the media/diluent and the center portion of the lens is at the liquid/air interface. Plates were loosely sealed to prevent evaporation, and incubated for 24 hours at 37° C, with rotary agitation. After incubation, each lens was subjected to the bacterial wash step as described in Example 5. Bacteria were recovered from the washed lenses and log kill calculated as described above in Example 5. Each t-24 hr inoculum concentration was confirmed as described above for t=0 and t=2 hr time points.

The antimicrobial effects of the 0 hour wash and 168 hour wash time point lenses are shown in Table 5:

**Table 5.**

| | Species | Log | | Log Reduction |
|---|---|---|---|---|
| | | Ctrl | εPLL | |
| **0 hr** | PA | 5.8 | 0.9 | 5.0 |
| | SM | 4.5 | 1.0 | 3.5 |
| | SA | 4.5 | 0.8 | 3.7 |
| **168 hr** | PA | 5.5 | 0.6 | 4.9 |
| | SM | 5.5 | 3.8 | 1.7 |
| | SA | 5.5 | 3.1 | 2.4 |

Table 5 demonstrates that lenses tested at the 168 hour time point exhibit bioactivity against the bacterial species tested. These lenses release only about 1 μg εPLL over a 24-hour period.

**Example 7: εPLL and PQ-1 are synergistic**

Lenses of Formulation F, prepared according to Example 1, were transferred to 6 ml glass vials containing containing 1.2 ml PBS either with 500 ppm εPLL (test lenses) or without εPLL (control lenses), sealed and autoclaved. The lenses were removed from their vials and placed in individual wells of a 24-well plate containing PBS or PBS plus PQ-1 at a concentration of 10 μg/ml (for both test and control lenses) or, for control lenses only, 30 μg/ml or 100 μg/ml. The plates were incubated for 72 hours at room temperature.

Lenses prepared in PBS without εPLL were tested using the bioactivity assay described in Example 5 using high inoculums ($10^7$). Table 6 below shows the log kill achieved by PQ-1 at three different concentrations after a 24-hour incubation with the bacteria.

**Table 6**

| Organism | log kill at 10 μg/ml PQ-1 | log kill at 30 μg/ml PQ-1 | log kill at 100 μg/ml PQ-1 |
|---|---|---|---|
| PA | -0.23 | 0.16 | 3.43 |
| SA | -0.54 | 1.47 | 3.04 |
| SM | -0.55 | 0.55 | 0.31 |

The lack of antimicrobial activity of the lenses packaged in PBS containing 30μg/ml PQ-1 against PA and SM was confirmed using an *in vitro* biofilm assay described in Example 6.

On the other hand, εPLL-containing lenses that were incubated with 10 μg/ml PQ-1 had an apparent synergistic antimicrobial effect against SA within 2 hours, which continued at 6 and 24 hours. Similar enhanced antimicrobial activity was seen against SM at 6 and 24 hours. The assay did not demonstrate enhanced activity against PA because εPLL alone is very effective against this organism, even at early time points. Table 7 shows the log kill for each individual lens and antimicrobial compound, as well as the combination of εPLL and PQ-1. Since the combined effect is more than the sum of the log kill for each compound separately, the antimicrobial action between PQ-1 and εPLL is considered synergistic, at least against SA and SM.

**Table 7**

| A | B | C | D | E | F |
|---|---|---|---|---|---|
| **Organism** | **Time (hr)** | **log kill 10 μ/ml PQ-1** | **log kill εPLL** | **predicted log kill PQ-1+εPLL** | **actual log kill PQ-1+εPLL** |
| PA | 2 | -- | 2.63 | -- | 2.37 |
| PA | 6 | -- | 4.04 | -- | 3.30 |
| PA | 24 | -0.23 | ≥ 4.04 | 3.81 | ≥ 4.04 |
| SA | 2 | -- | -0.06 | -- | 1.45 |
| SA | 6 | -- | 0.37 | -- | 2.06 |
| SA | 24 | -0.74 | 1.85 | 1.11 | 2.64 |
| SM | 2 | -- | 0.19 | -- | 0.80 |
| SM | 6 | -- | 0.74 | -- | 1.18 |
| SM | 24 | 0.55 | 1.63 | 2.18 | 3.82 |

**Example 8: Affect of ionic strength of packaging solution on εPLL uptake by ionic silicone hydrogel contact lenses.**

Lenses of formulation F prepared according to Example 1 were transferred to 6 ml glass vials containing 1.2 ml 500 ppm εPLL in either PBS or deionized water. The vials were then sealed and autoclaved. As determined using the methods described in Example 2, the lenses packaged in PBS took up an average of 233 μg εPLL from the packaging solution, which represented 39% of the total εPLL available in the packaging solution. In surprising contrast, the lenses packaged in deionized water took up on average 575 μg εPLL, which represented 96% of the available εPLL. The study was repeated substituting the deionized water with the TRIS/sorbitol buffers shown in Tables 8 and 9 below:

**Table 8:** 19mM TRIS Buffer (pH 7.30) with 2% Sorbitol

| **Components** | **Target Wt. (g)** | **%** |
|---|---|---|
| TRIS (hydroxymethyl) Amino Methane ($C_4H_{11}NO_3$) | 0.23 | 0.02 |
| Trizma Hydrochloride ($C_4H_{11}NO_3.HCL$) | 2.75 | 0.27 |
| Sorbitol ($C_6H_{14}O_6$) | 20.00 | 1.96 |
| deionized $H_2O$ | 1000.00 | 97.75 |
| Total | 1022.98 | 100.00 |

**Table 9:** 19mM TRIS Buffer (pH 7.30) with 5% Sorbitol

| **Components** | **Target Wt. (g)** | **%** |
|---|---|---|
| TRIS (hydroxymethyl) Amino Methane ($C_4H_{11}NO_3$) | 0.23 | 0.02 |
| Trizma Hydrochloride ($C_4H_{11}NO_3.HCL$) | 2.75 | 0.26 |

(continued)

| Components | Target Wt. (g) | % |
|---|---|---|
| Sorbitol ($C_6H_{14}O_6$) | 50.00 | 4.75 |
| deionized $H_2O$ | 1000.00 | 94.97 |
| Total | 1052.98 | 100.00 |

On average, the lenses packaged in the TRIS/sorbitol buffers of Tables 8 and 9 took up 408 μg and 386 μg εPLL, respectively.

Although the disclosure herein refers to certain illustrated examples, it is to be understood that these examples are presented by way of example and not by way of limitation. The intent of the foregoing detailed description, although discussing exemplary examples, is to be construed to cover all modifications, alternatives, and equivalents of the examples as may fall within the spirit and scope of the invention as defined by the claims.

The invention further provides:

1. A sealed package containing an antimicrobial contact lens immersed in a packaging solution, said contact lens comprising a hydrogel and an antimicrobially-effective amount of epsilon polylysine (εPLL). Optionally, the epsilon polylysine is non-covalently attached to the hydrogel.
2. The package of 1, wherein the hydrogel comprises silicone.
3. The package of 1 or 2, wherein the hydrogel comprises negatively charged groups. The negatively charged groups advantageously ionically bind to the εPLL.
4. The package of any one of 1 to 3, wherein the hydrogel comprises a hydrated polymerization product of a monomer mixture that comprises an anionic monomer.
5. The package of 4, wherein the hydrogel has an ionic content of about 0.5% to about 2.5%, the ionic content being the sum of the % ionic content provided by each anionic monomer **n** as determined by Formula I:

$$\sum (\boldsymbol{a_n}\ x\ \boldsymbol{b_n} / \mathbf{c}_\mathrm{n})\ \mathrm{x}\ 89 = \%\ \text{ionic content} \qquad \mathbf{(I)}$$

where $\boldsymbol{a_n}$ is the weight percentage of the anionic monomer **n** in the monomer mixture relative to the weight of all components of the monomer mixture that incorporate into the hydrogel, $\boldsymbol{b_n}$ is the number of negatively-charged groups on monomer **n** at pH 7 (for example, the number of carboxylate, phosphate, phosphonate, phosphonic, sulfonate, sulfate and sulfite groups in the monomer), and $\boldsymbol{c_n}$ is the molecular weight of ionic monomer **n.**

6. The package of 4, wherein the hydrogel has an ionic content of about 1.5% to about 2.2%.
7. The package of 4, wherein the hydrogel has an ionic content of about 1.6% to about 2.0%.
8. The package of 1 to 7, wherein the hydrogel comprises phosphorylcholine groups. The phosphorylcholine groups advantageously ionically bind to the εPLL.
9. The package of 1 to 8, wherein the packaging solution has an ionic strength of less than about 0.15.
10. The package of 1 to 9, wherein the contact lens comprises at least 10 μg εPLL.
11. The package of 1 to 9, wherein the contact lens 1 comprises at least 100 μg εPLL.
12. A method of replenishing or enhancing antimicrobial activity of a contact lens after it has been worn, comprising storing the worn contact lens in a multi-purpose contact lens care solution (MPS) that comprises additional εPLL and/or a second antimicrobial agent, wherein additional εPLL and/or second antimicrobial agent incorporates into the contact lens during the storage. The contact lens may, for example, be as defined in any one of 1 to 8, 10 or 11 above.
13. A method of manufacturing an unworn antimicrobial contact lens, said method comprising: polymerizing a monomer mixture to provide a lens-shaped polymerization product; optionally hydrating the polymerization product to form a hydrogel; immersing the polymerization product or hydrogel in a package containing a packaging solution; sealing the package; and optionally sterilizing the sealed package by autoclave, wherein an antimicrobially effective amount of εPLL is non-covalently attached to the hydrogel.
14. The method of 13, wherein the polymerization product has an ionic content of about 1 to about 2%, and wherein the packaging solution comprises from about 100 ppm to about 1000 ppm εPLL.
15. A method of manufacturing a packaged antimicrobial hydrogel contact lens including the step of bringing a lens-shaped polymerization product or a hydrogel contact lens into contact with a packaging solution that comprises εPLL.
16. The method of 15 wherein the packaging solution comprises from about 50 ppm to about 10,000 ppm εPLL, especially from about 100 ppm to about 600 ppm εPLL.

17. The method of 15 or 16 wherein the hydrogel of the packaged antimicrobial hydrogel contact lens is as defined in any one of 2 to 8.

1 a. An unworn antimicrobial contact lens immersed in a packaging solution and sealed in a package, said contact lens comprising a hydrogel and an antimicrobially-effective amount of epsilon polylysine (εPLL) non-covalently attached to the hydrogel.
2a. The contact lens of 1a, wherein the hydrogel comprises silicone.
3a. The contact lens of 1a or 2a, wherein the hydrogel comprises negatively charged groups that ionically bind to the εPLL.
4a. The contact lens of any of 1a to 3a, wherein the hydrogel comprises a hydrated polymerization product of a monomer mixture that comprises an anionic monomer.
5a. The contact lens of any of 1a to 4a, wherein the hydrogel has an ionic content of about 0.5% to about 1.5%.
6a. The contact lens of any of 1a to 5a, wherein the hydrogel has an ionic content of about 1.5% to about 2.2%.
7a. The contact lens of any of 1a to 6a, wherein the hydrogel has an ionic content of about 1.6% to about 2.0%.
8a. The contact lens of any of 1a to 7a, wherein the hydrogel comprises phosphorylcholine groups that ionically bind to the εPLL.
9a. The contact lens of any of 1a to 7a, wherein the packaging solution comprises from about 50 ppm to about 10,000 ppm εPLL prior to contact with the hydrogel.
10a. The contact lens of 9a, wherein the packaging solution comprises from about 100 ppm to about 600 ppm εPLL prior to contact with the hydrogel.
11a. The contact lens of any of 1a to 10a, wherein the packaging solution has an ionic strength of less than about 0.15.
12a. The contact lens of any of 1a to 11a, that results in at least a four log kill of *Pseudomonas auruginosa* when tested in an *in vitro* bioactivity assay using a $10^4$ inoculum and a 24-hour incubation.
13a. The contact lens of any of 1a to 12a, that results in at least a two log kill of *Serratia marcescens* and/or *Staphylococcus aureus.*
14a. The contact lens of any of 1a to 13a, that results in at least a two log kill of *Pseudomonas auruginosa* after 24 hours of *in vitro* εPLL release.
15a. The contact lens of any of 1a to 14a, that results in at least a two log kill of *Pseudomonas auruginosa* after 48 hours of *in vitro* εPLL release.
16a. The contact lens of any of 1a to 15a, comprising at least 10 μg εPLL.
17a. The contact lens of 16a, comprising at least 100 μg εPLL.
18a. The contact lens of any of 1a to 17a, wherein the hydrogel sustains release of εPLL for at least 2 hours.
19a. The contact lens of 18a, wherein the hydrogel sustains release of εPLL for at least 12 hours.
20a. The contact lens of any of 1a to 19a, which is an extended wear contact lens suitable to be worn continuously by a patient for at least 5 days.
21a. A method of replenishing or enhancing antimicrobial activity of the contact lens of any of 1a to 20a after it has been worn, comprising storing the worn contact lens in a multi-purpose contact lens care solution (MPS) that comprises additional εPLL and/or a second antimicrobial agent, wherein additional εPLL and/or second antimicrobial agent incorporates into the contact lens during the storage.
22a. The method of 21a, wherein the MPS comprises polyquaternium-1.
23a. A method of manufacturing an unworn antimicrobial contact lens, said method comprising: polymerizing a monomer mixture to provide a lens-shaped polymerization product; optionally hydrating the polymerization product to form a hydrogel; immersing the polymerization product or hydrogel in a package containing a packaging solution; sealing the package; and optionally sterilizing the sealed package by autoclave, wherein an antimicrobially effective amount of εPLL is non-covalently attached to the hydrogel.
24a. The method of 23a, wherein the polymerization product has an ionic content of about 1 to about 2%, and wherein the packaging solution comprises from about 100 ppm to about 1000 ppm εPLL.
25a. A method of manufacturing a packaged antimicrobial hydrogel contact lens including the step of bringing a lens-shaped polymerization product or a hydrogel contact lens into contact with a packaging solution that comprises εPLL.
26a. The method of 25a, wherein the packaging solution comprises from about 50 ppm to about 10,000 ppm εPLL, especially from about 100 ppm to about 600 ppm εPLL.
27a. The method of 25a or 26a, wherein the hydrogel of the packaged antimicrobial hydrogel contact lens is as defined in any one of 2 a to 8 a.
28a. A composition for use in the treatment or prophylaxis of a microbial infection, comprising a hydrogel and epsilon polylysine (εPLL) non-covalently attached to the hydrogel.
29a. The composition of 28a, as further defined in any one of 1a to 8a or 12a to 19a. 30a. The composition of 28a or 29a, in the form of a contact lens.

31a. The composition of any one of 28a to 30a immersed in a packing solution and sealed in a package.
32a. A method of administering epsilon polylysine (εPLL) to the ocular tissue of a patient in need thereof, said method comprising administering the patient the contact lens of any one of 1 a to 20a.

## Claims

1. A method of replenishing or enhancing antimicrobial activity of a contact lens after it has been worn, comprising storing the worn contact lens in a multi-purpose contact lens care solution (MPS) that comprises additional εPLL and/or a second antimicrobial agent, wherein additional εPLL and/or second antimicrobial agent incorporates into the contact lens during the storage.

2. The method of claim 1, wherein the contact lens comprises a hydrogel.

3. The method of claim 2, wherein the hydrogel comprises (a) silicone; (b) negatively charged groups that ionically bind to the εPLL; and/or (c) phosphorylcholine groups that ionically bind to the εPLL.

4. The method of claim 2 or claim 3, wherein (a) the hydrogel comprises a hydrated polymerization product of a monomer mixture that comprises an anionic monomer; and/or (b) the hydrogel has an ionic content of 0.5% to 1.5%, 1.5% to 2.2% or 1.6% to 2.0%.

5. The method of any one of claims 2 to 4, wherein the hydrogel sustains release of εPLL for at least 2 hours, especially for at least 12 hours.

6. The method of any preceding claim, wherein the contact lens results in (a) at least a four log kill of *Pseudomonas auruginosa* when tested in an *in vitro* bioactivity assay using a $10^4$ inoculum and a 24-hour incubation; (b) at least a two log kill of *Serratia marcescens* and/or *Staphylococcus aureus*; (c) at least a two log kill of *Pseudomonas auruginosa* after 24 hours of *in vitro* εPLL release; and/or (d) at least a two log kill of *Pseudomonas auruginosa* after 48 hours of *in vitro* εPLL release.

7. The method of any preceding claim, wherein the contact lens comprises at least 10 μg εPLL, especially at least 100 μg εPLL.

8. The method of any preceding claim, wherein the contact lens is an extended wear contact lens suitable to be worn continuously by a patient for at least 5 days.

9. The method of any preceding claim, wherein the MPS comprises polyquaternium-1.

10. A composition for use in the treatment or prophylaxis of a microbial infection, comprising a hydrogel and epsilon polylysine (εPLL) non-covalently attached to the hydrogel.

11. The composition of claim 10, wherein the hydrogel is as further defined in any one of claims 3 to 5.

12. The composition of claim 10 or claim 11, in the form of a contact lens, wherein the contact lens is optionally as further defined in any one of claims 6 to 8.

13. The composition of any one of claims 10 to 12 immersed in a packing solution and sealed in a package, wherein the packaging solution optionally (a) comprises from 50 ppm to 10,000 ppm εPLL prior to contact with the hydrogel, especially from 100 ppm to 600 ppm εPLL prior to contact with the hydrogel and/or (b) has an ionic strength of less than 0.15.

14. The composition of any one of claims 10 to 13 for use in administering epsilon polylysine (εPLL) to the ocular tissue of a patient in need thereof.

15. A sealed package containing an antimicrobial contact lens immersed in a packaging solution, said contact lens comprising a hydrogel and an antimicrobially-effective amount of epsilon polylysine (εPLL), wherein optionally, the epsilon polylysine is non-covalently attached to the hydrogel.

**16.** The sealed package of claim 15, wherein the hydrogel is as defined in any one of claims 3 to 5, the contact lens is as defined in any one of claims 6 to 8, and/or wherein the packaging solution is as defined in claim 13.

**17.** A method of manufacturing an unworn antimicrobial contact lens, said method comprising:

polymerizing a monomer mixture to provide a lens-shaped polymerization product; optionally hydrating the polymerization product to form a hydrogel; immersing the polymerization product or hydrogel in a package containing a packaging solution; sealing the package; and
optionally sterilizing the sealed package by autoclave, wherein an antimicrobially effective amount of εPLL is non-covalently attached to the hydrogel.

**18.** The method of claim 16, wherein the polymerization product has an ionic content of 1 to 2%, and wherein the packaging solution comprises from 100 ppm to 1000 ppm εPLL.

Europäisches Patentamt
European Patent Office
Office européen des brevets

# EUROPEAN SEARCH REPORT

Application Number
EP 16 17 9554

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 884 058 A1 (ASAHI CHEMICAL IND [JP]) 16 December 1998 (1998-12-16) * page 3, line 9 - line 39; example 2 * | 1-18 | INV. G02B1/04 G02C7/04 A61F9/00 |
| X | WO 2011/149058 A1 (UNIV NANYANG TECH [SG]; MENICON CO LTD [JP]) 1 December 2011 (2011-12-01) * claims 1,6 * | 15,16 | |
| X | EP 1 473 584 A1 (OPHTECS CORP [JP]) 3 November 2004 (2004-11-03) * paragraph [0030] - paragraph [0031] * | 1,2, 9-11,14, 15,17,18 | |
| A | JP 2001 278984 A (KURARAY CO) 10 October 2001 (2001-10-10) * abstract * | 1 | |

TECHNICAL FIELDS SEARCHED (IPC)
G02B
G02C
A61F

The present search report has been drawn up for all claims

1

| Place of search | Date of completion of the search | Examiner |
|---|---|---|
| Munich | 28 February 2017 | Kukolka, Florian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 9554

This annex lists the patent family members relating to the patent documents cited in the above-mentioned European search report. The members are as contained in the European Patent Office EDP file on
The European Patent Office is in no way liable for these particulars which are merely given for the purpose of information.

28-02-2017

| Patent document cited in search report | Publication date | Patent family member(s) | Publication date |
|---|---|---|---|
| EP 0884058 A1 | 16-12-1998 | CA 2245515 A1 | 07-08-1997 |
| | | CN 1212631 A | 31-03-1999 |
| | | DE 69723236 D1 | 07-08-2003 |
| | | DE 69723236 T2 | 12-02-2004 |
| | | EP 0884058 A1 | 16-12-1998 |
| | | ES 2198547 T3 | 01-02-2004 |
| | | US 6187264 B1 | 13-02-2001 |
| | | WO 9727879 A1 | 07-08-1997 |
| WO 2011149058 A1 | 01-12-2011 | CN 103298495 A | 11-09-2013 |
| | | EP 2578244 A1 | 10-04-2013 |
| | | JP 2011245053 A | 08-12-2011 |
| | | KR 20130079432 A | 10-07-2013 |
| | | SG 185757 A1 | 28-12-2012 |
| | | SG 10201504171X A | 30-07-2015 |
| | | US 2014163133 A1 | 12-06-2014 |
| | | WO 2011149058 A1 | 01-12-2011 |
| EP 1473584 A1 | 03-11-2004 | CA 2443472 A1 | 14-08-2003 |
| | | CN 1498354 A | 19-05-2004 |
| | | EP 1473584 A1 | 03-11-2004 |
| | | JP 4255839 B2 | 15-04-2009 |
| | | KR 20040080923 A | 20-09-2004 |
| | | TW I268784 B | 21-12-2006 |
| | | US 2005074467 A1 | 07-04-2005 |
| | | WO 03067311 A1 | 14-08-2003 |
| JP 2001278984 A | 10-10-2001 | JP 4044291 B2 | 06-02-2008 |
| | | JP 2001278984 A | 10-10-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6187264 B
- US 20050074467 A
- US 20070149428 A
- US 20090100801 A
- US 20120074352 A
- US 20110071091 A
- US 20040135967 A
- US 4168112 A
- US 7282214 B
- US 7402318 B
- EP 1328303 B1
- WO 9413774 A
- US 6867245 B, Iwata
- US 8129442 B, Ueyama
- US 4889664 A, Kindt-Larsen
- US 3630200 A, Higuchi
- US 6217896 B, Benjamin
- US 4143949 A, Chen
- US 20080245474 A, Claude
- US 8168735 B, Ichinohe


### Non-patent literature cited in the description


- **ZHOU et al.** *Biomaterials,* 2011, vol. 32, 2704-2712
- *CHEMICAL ABSTRACTS,* 28211-04-3
- United States Pharmacopeia 32 / National Formulary 27. *United States Pharmacopeial Convention,* 2009
- *CHEMICAL ABSTRACTS,* 96478-09-0
- *CHEMICAL ABSTRACTS,* 40538-11-2
- *CHEMICAL ABSTRACTS,* 109561-07-1
- *CHEMICAL ABSTRACTS,* 78-67-1